# EUROPEAN PATENT APPLICATION

(11) **EP 4 471 026 A1**
(43) Date of publication of application: **04.12.2024**
(21) Application number: 23746088.6
(22) Date of filing: 16.01.2023
(51) Int. Cl.: C07D 405/14, C07D 405/02, C07D 405/00, C07D 403/00

(54) **COUMARIN COMPOUNDS AND USES THEREOF**

(30) Priority: 26.01.2022 CN 202210093816; 18.04.2022 CN 202210405730
(71) Applicant: Medshine Discovery Inc., Nanjing, Jiangsu 210032 (CN)
(72) Inventor: ZHANG, Dongkai, Shanghai 200131 (CN); TAN, Haizhong, Shanghai 200131 (CN); WU, Wentao, Shanghai 200131 (CN); XU, Yangyang, Shanghai 200131 (CN); SUN, Jikui, Shanghai 200131 (CN); GAO, Na, Shanghai 200131 (CN); ZHANG, Yang, Shanghai 200131 (CN); LI, Jian, Shanghai 200131 (CN); CHEN, Shuhui, Shanghai 200131 (CN)
(74) Representative: Cabinet Becker et Associés
(86) International application number: PCT/CN2023/072434
(87) International publication number: WO 2023/143206

(57) **Abstract**

Disclosed in the present invention are a series of coumarin compounds and the uses thereof, and particularly disclosed are compounds as shown in formula (I) and pharmaceutically acceptable salts thereof.

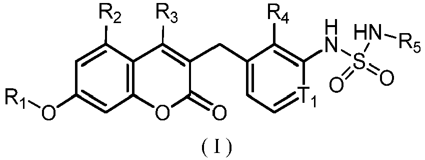

## Description

This application claims the priority of:
CN202210093816.8, filed on January 26, 2022;
CN202210405730.4, filed on April 18, 2022.

### TECHNICAL FIELD

The present disclosure discloses a series of coumarin compounds and applications thereof, and specifically discloses a compound represented by formula (I) and a pharmaceutically acceptable salt thereof.

### BACKGROUND

The mitogen-activated protein kinase (MAPK) signaling pathway is one of the important pathways in the signal transduction network of eukaryotic organisms. It is a key signaling pathway for cell proliferation, differentiation, apoptosis, and stress response under normal and pathological conditions. MAPK is a group of evolutionarily conserved serine-threonine kinases. The RAS/RAF/MEK/ERK signaling pathway is one of the MAPK pathways. Its protein overexpression or mutation has been found in many malignant tumors. The RAS/RAF/MEK/ERK signaling pathway is one of the typical signaling pathways in the field of tumors.

Statistics show that 31% of non-small cell lung cancer in patients is driven by KRAS mutations, 90% of pancreatic cancer in patients is driven by KRAS mutations; in addition, 21 % of endometrial cancer, 45% of colorectal cancer, 5% of ovarian cancer, etc. are all driven by KRAS mutations; 28% of melanoma and 20% of multiple myeloma are driven by NRAS mutations; 60% of melanoma, 35-60% of ovarian cancer, 30-80% of papillary thyroid cancer, etc. are driven by BRAF mutations. There is a large population of patients.

There are already several drugs targeting RAF and MEK on the market, but the approved indications of the currently marketed RAF small molecule inhibitors and MEK small molecule inhibitors are concentrated in melanoma with BRAF^{V600E} mutation. Under normal circumstances, BRAF has kinase activity only after dimerization after being phosphorylated by RAS, while mutated BRAF is always in an activated state. A BRAF^{V600E} monomer can phosphorylate MEK. In the case of RAS mutations, BRAF and CRAF form heterodimers to exert their effects. Existing RAF and MEK drugs are only effective for BRAF^{V600E} mutation, and are ineffective for tumors with RAS mutations upstream of MAPK. There is a need for new second-generation RAF small molecule inhibitors. The MEK inhibitors that are also on the market are allosteric kinase inhibitors. Although allosteric inhibitors are highly specific for MEK, they are susceptible to upstream kinase signals. Feedback regulation of MEK phosphorylation can cause the RAS pathway signal to be reactivated, and inhibiting MEK alone cannot effectively treat solid tumors with RAS mutations. Therefore, new dual RAF/MEK inhibitors may become an option for treating diseases with MAPK pathway activation.

### SUMMARY

The present disclosure provides a compound represented by formula (I) or a pharmaceutically acceptable salt thereof 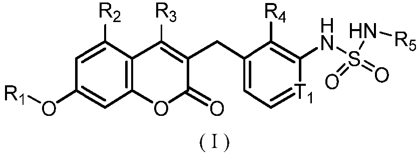 wherein
T₁ is selected from CH and N;
R₁ is selected from pyrimidinyl and -C(=O)-C₁₋₄ alkylamino;
R₂ is halogen;
R₃ is selected from halogen, C₁₋₃ alkyl, -CH₂-C₁₋₄ alkylamino and -CH₂-4- to 6-membered N-containing heterocycloalkyl, wherein the C₁₋₃ alkyl, -CH₂-C₁₋₄ alkylamino and -CH₂-4- to 6-membered N-containing heterocycloalkyl are each independently optionally substituted with 1, 2 or 3 halogens;
R₄ is selected from halogen and C₁₋₃ alkyl, wherein the C₁₋₃ alkyl is optionally substituted with 1, 2 or 3 halogens;
R₅ is selected from C₁₋₃ alkyl, wherein the C₁₋₃ alkyl is optionally substituted with 1, 2 or 3 halogens.

In some embodiments of the present disclosure, the R₁ is selected from 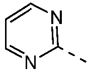 and 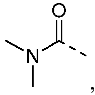 and other variables are as defined in the present disclosure.

In some embodiments of the present disclosure, the R₂ is F, and other variables are as defined in the present disclosure.

In some embodiments of the present disclosure, the R₃ is selected from F, Cl, Br, CH₃, CH₂CH₃, CH₂NHCH₃ and CH₂N(CH₃)₂, wherein the CH₃, CH₂CH₃, CH₂NHCH₃ and CH₂N(CH₃)₂ are optionally substituted with 1, 2 or 3 halogens, and other variables are as defined in the present disclosure.

In some embodiments of the present disclosure, the R₃ is selected from CH₃ and CH₂N(CH₃)₂, and other variables are as defined in the present disclosure.

In some embodiments of the present disclosure, the R₄ is F, and other variables are as defined in the present disclosure.

In some embodiments of the present disclosure, the R₅ is selected from CH₃, and other variables are as defined in the present disclosure.

Some other embodiments of the present disclosure are obtained by arbitrarily combining the above variables.

The present disclosure also provides a compound represented by the following formula or a pharmaceutically acceptable salt thereof:

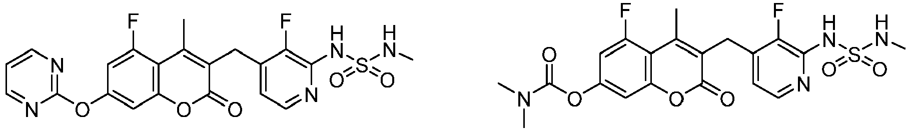

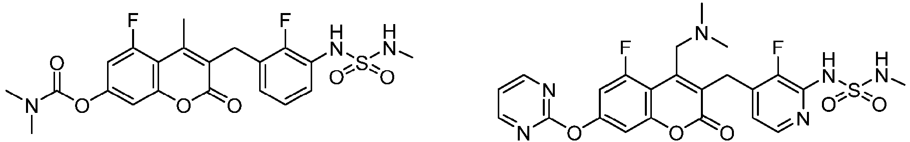

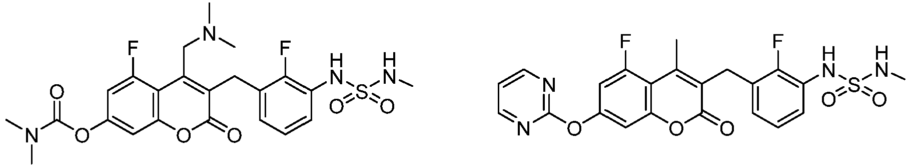

### Technical Effects

The compounds of the present disclosure can inhibit MEK target and RAF target, can effectively inhibit the proliferation of HCT116 cells, and have high unbound plasma exposure, good oral bioavailability and significant tumor inhibition effect in mice.

### Related Definitions

Unless otherwise specified, the following terms and phrases used herein are intended to have the following meanings. A specific term or phrase should not be considered indefinite or unclear in the absence of a particular definition, but should be understood in the conventional sense. When a trade name appears herein, it is intended to refer to its corresponding commodity or active ingredient thereof.

The term "pharmaceutically acceptable" is used herein in terms of those compounds, materials, compositions, and/or dosage forms, which are suitable for use in contact with human and animal tissues within the scope of reliable medical judgment, with no excessive toxicity, irritation, allergic reaction or other problems or complications, commensurate with a reasonable benefit/risk ratio.

The term "pharmaceutically acceptable salt" means a salt of compounds disclosed herein that is prepared by reacting the compound having a specific substituent disclosed herein with a relatively non-toxic acid or base. When compounds disclosed herein contain a relatively acidic functional group, a base addition salt can be obtained by bringing the compound into contact with a sufficient amount of base in a pure solution or a suitable inert solvent. The pharmaceutically acceptable base addition salt includes a salt of sodium, potassium, calcium, ammonium, organic amine or magnesium or similar salts. When compounds disclosed herein contain a relatively basic functional group, an acid addition salt can be obtained by bringing the compound into contact with a sufficient amount of acid in a pure solution or a suitable inert solvent. Examples of the pharmaceutically acceptable acid addition salt include an inorganic acid salt, wherein the inorganic acid includes, for example, hydrochloric acid, hydrobromic acid, nitric acid, carbonic acid, bicarbonate, phosphoric acid, monohydrogen phosphate, dihydrogen phosphate, sulfuric acid, hydrogen sulfate, hydroiodic acid, phosphorous acid, and the like; and an organic acid salt, wherein the organic acid includes, for example, acetic acid, propionic acid, isobutyric acid, maleic acid, malonic acid, benzoic acid, succinic acid, suberic acid, fumaric acid, lactic acid, mandelic acid, phthalic acid, benzenesulfonic acid, p-toluenesulfonic acid, citric acid, tartaric acid, and methanesulfonic acid, and the like; and an salt of amino acid (such as arginine and the like), and a salt of an organic acid such as glucuronic acid and the like. Certain specific compounds disclosed herein contain both basic and acidic functional groups and can be converted to any base or acid addition salt.

The pharmaceutically acceptable salt disclosed herein can be prepared from the parent compound that contains an acidic or basic moiety by conventional chemical methods. Generally, such salt can be prepared by reacting the free acid or base form of the compound with a stoichiometric amount of an appropriate base or acid in water or an organic solvent or a mixture thereof.

Compounds disclosed herein may be present in a specific geometric or stereoisomeric form. The present disclosure contemplates all such compounds, including cis and trans isomers, (-)- and (+)-enantiomers, (R)- and (S)-enantiomers, diastereoisomer, (D)-isomer, (L)-isomer, and a racemic mixture and other mixtures, for example, a mixture enriched in enantiomer or diastereoisomer, all of which are encompassed within the scope disclosed herein. The substituent such as alkyl may have an additional asymmetric carbon atom. All these isomers and mixtures thereof are encompassed within the scope disclosed herein.

Compounds disclosed herein may contain an unnatural proportion of atomic isotopes at one or more of the atoms that make up the compounds. For example, a compound may be labeled with a radioisotope such as tritium (³H), iodine-125 (¹²⁵I) or C-14(¹⁴C). For another example, hydrogen can be replaced by heavy hydrogen to form a deuterated drug. The bond between deuterium and carbon is stronger than that between ordinary hydrogen and carbon. Compared with undeuterated drugs, deuterated drugs have advantages of reduced toxic side effects, increased drug stability, enhanced efficacy, and prolonged biological half-life of drugs. All changes in the isotopic composition of compounds disclosed herein, regardless of radioactivity, are included within the scope of the present disclosure.

The term "optional" or "optionally" means that the subsequent event or condition may occur but not requisite, that the term includes the instance in which the event or condition occurs and the instance in which the event or condition does not occur.

The term "substituted" means that one or more than one hydrogen atoms on a specific atom are substituted by a substituent, including deuterium and hydrogen variants, as long as the valence of the specific atom is normal and the substituted compound is stable. When the substituent is oxo (i.e., =O), it means two hydrogen atoms are substituted. Positions on an aromatic ring cannot be substituted by oxo. The term "optionally substituted" means an atom can be substituted by a substituent or not, unless otherwise specified, the species and number of the substituent may be arbitrary so long as being chemically achievable.

When any variable (such as R) occurs in the constitution or structure of the compound more than once, the definition of the variable at each occurrence is independent. Thus, for example, if a group is substituted by 0-2 R, the group can be optionally substituted by up to two R, wherein the definition of R at each occurrence is independent. Moreover, a combination of the substituent and/or the variant thereof is allowed only when the combination results in a stable compound.

When the number of a linking group is 0, such as -(CRR)₀-, it means that the linking group is a single bond.

When one of variables is a single bond, it means that the two groups linked by the single bond are connected directly. For example, when L in A-L-Z represents a single bond, the structure of A-L-Z is actually A-Z.

When an enumerated linking group does not indicate its linking direction, its linking direction is arbitrary. For example, when the linking group L in 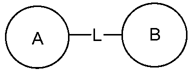 is -M-W-, the -M-W- can be linked to the ring A and the ring B in the same direction as the reading order from left to right to constitute 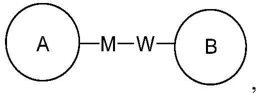 or can be linked to the ring A and the ring B in the reverse direction as the reading order from left to right to constitute 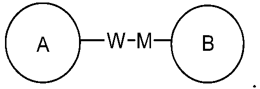 A combination of the linking groups, substituents and/or variants thereof is allowed only when such combination can result in a stable compound.

Unless otherwise specified, when a group has one or more connectable sites, any one or more sites of the group can be connected to other groups through chemical bonds. Where the connection position of the chemical bond is variable, and there is H atom(s) at a connectable site(s), when the connectable site(s) having H atom(s) is connected to the chemical bond, the number of H atom(s) at this site will correspondingly decrease as the number of the connected chemical bond increases, and the group will become a group of corresponding valence. The chemical bond between the site and other groups can be represented by a straight solid bond 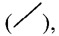 a straight dashed bond 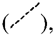 or a wavy line 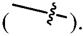 For example, the straight solid bond in -OCH₃ indicates that the group is connected to other groups through the oxygen atom in the group; the straight dashed bond in 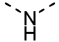 indicates that the group is connected to other groups through two ends of the nitrogen atom in the group; the wavy line in 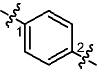 indicates that the group is connected to other groups through the 1- and 2-carbon atoms in the phenyl group; 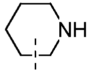 indicates that any connectable site on the piperidinyl group can be connected to other groups through one chemical bond, including at least four connection ways, 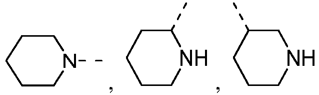 and 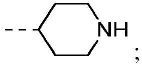 even if a H atom is drawn on -N-, 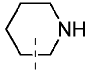 still includes the connection way of 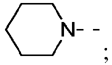 it's just that when one chemical bond is connected, the H at this site will be reduced by one, and the group will become the corresponding monovalent piperidinyl group.

Unless otherwise specified, a wedged solid bond ( 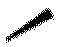 ) and a wedged dashed bond ( 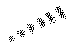 ) indicate the absolute configuration of a stereocenter; a straight solid bond ( 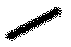 ) and a straight dashed bond ( 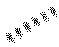 ) indicate the relative configuration of a stereocenter; a wavy line ( 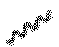 ) indicates a wedged solid bond ( 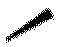 ) or a wedged dashed bond ( 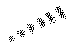 ); or a wavy line ( 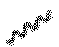 ) indicates a straight solid bond ( 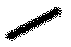 ) or a straight dashed bond ( 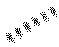 ).

Unless otherwise specified, the term "halo" or "halogen" by itself or as part of another substituent means a fluorine (F), chlorine (Cl), bromine (Br) or iodine (I) atom.

Unless otherwise specified, the term "C₁₋₃ alkyl" is used to represent a linear or branched saturated hydrocarbon group composed of 1 to 3 carbon atoms. The C₁₋₃ alkyl includes C₁₋₂ alkyl, C₂₋₃ alkyl, etc. It may be monovalent (such as methyl), divalent (such as methylene) or multivalent (such as methenyl). Examples of the C₁₋₃ alkyl include, but are not limited to, methyl (Me), ethyl (Et), propyl (including n-propyl and isopropyl), and the like.

Unless otherwise specified, the term "C₁₋₄ alkylamino" means alkyl groups containing 1 to 4 carbon atoms and attached to the remainder of a molecule by an amino group. The C₁₋₄ alkylamino group includes C₁₋₃, C₁₋₂, C₂₋₄, C₄, C₃ and C₂ alkylamino groups and the like. Examples of C₁₋₄ alkylamino groups include, but are not limited to -NHCH₃, -N(CH₃)₂, -NHCH₂CH₃, - N(CH₃)CH₂CH₃, -N(CH₂CH₃)(CH₂CH₃), -NHCH₂CH₂CH₃, -NHCH₂(CH₃)₂, - NHCH₂CH₂CH₂CH₃, and the like.

The term "4- to 6-membered N-containing heterocycloalkyl" alone or in combination with other terms respectively represents a saturated cyclic group composed of 4 to 6 ring atoms, in which 1, 2, 3 or 4 ring atoms are heteroatoms independently selected from O, S and N, at least one heteroatom is a N atom, and the remainder is carbon atoms, wherein the nitrogen atom is optionally quaternized, and the nitrogen and sulfur heteroatoms are optionally oxidized (i.e., NO and S(O)ₚ, p is 1 or 2). It includes monocyclic and bicyclic ring systems, wherein the bicyclic ring system includes spirocyclic, fused cyclic and bridged rings. In addition, with respect to the "4- to 6-membered N-containing heterocycloalkyl", a heteroatom may be present on the position of attachment of the heterocycloalkyl group to the remainder of a molecule. The 4- to 6-membered heterocycloalkyl includes 5- to 6- membered, 4 membered, 5 membered and 6 membered heterocycloalkyl, etc. Examples of the 4- to 6-membered heterocycloalkyl include, but are not limited to, azetidinyl, pyrrolidinyl, pyrazolidinyl, imidazolidinyl, piperidinyl (including 1-piperidinyl, 2-piperidinyl and 3-piperidinyl, etc.), piperazinyl (including 1-piperazinyl and 2-piperazinyl, etc.), morpholinyl (including 3-morpholinyl and 4-morpholinyl, etc.), hexahydropyridazinyl, and the like.

Unless otherwise specified, Cₙ₋ₙ₊ₘ or Cₙ-Cₙ₊ₘ includes any specific case of n to n+m carbons, for example, C₁₋₁₂ includes C₁, C₂, C₃, C₄, C₅, C₆, C₇, C₈, C₉, C₁₀, C₁₁ and C₁₂, also includes any range from n to n+m, for example, C₁₋₁₂ includes C₁₋₃, C₁₋₆, C₁₋₉, C₃₋₆, C₃₋₉, C₃₋₁₂, C₆₋₉, C₆₋₁₂ and C₉₋₁₂, etc.; similarly, n membered to n+m membered indicates that the number of atoms on a ring is n to n+m, for example, 3-12 membered ring includes 3 membered ring, 4 membered ring, 5 membered ring, 6 membered ring, 7 membered ring, 8 membered ring, 9 membered ring, 10 membered ring, 11 membered ring, and 12 membered ring, also includes any range from n to n+m, for example, 3-12 membered ring includes 3-6 membered ring, 3-9 membered ring, 5-6 membered ring, 5-7 membered ring, 6-7 membered ring, 6-8 membered ring, and 6-10 membered ring, and the like.

Compounds disclosed herein can be prepared by a variety of synthetic methods well known to those skilled in the art, including the following enumerated embodiment, the embodiment formed by the following enumerated embodiment in combination with other chemical synthesis methods, and equivalent replacement well known to those skilled in the art. Alternative embodiments include, but are not limited to the embodiment disclosed herein.

The structures of compounds disclosed herein can be confirmed by conventional methods well known to those skilled in the art. If the present disclosure relates to an absolute configuration of a compound, the absolute configuration can be confirmed by conventional techniques in the art, such as single crystal X-Ray diffraction (SXRD). In the single crystal X-Ray diffraction (SXRD), the diffraction intensity data of the cultivated single crystal is collected using a Bruker D8 venture diffractometer with a light source of CuKα radiation in a scanning mode of ϕ/ω scan; after collecting the relevant data, the crystal structure is further analyzed by the direct method (Shelxs97) to confirm the absolute configuration.

Solvents used in the present disclosure are commercially available.

Compounds are named according to general naming principles in the art or by ChemDraw^{®} software, and commercially available compounds are named with their vendor directory names.

### BRIEF DESCRIPTION OF DRAWINGS

Fig. 1 is a graph showing the changes in tumor volume of mice at different dosages of compound 3 over 28 days;
Fig. 2 is a graph showing the changes in body weight of mice at different dosages of compound 3 over 28 days.

### DETAILED DESCRIPTION

The present disclosure is described in detail below by means of examples. However, it is not intended that these examples have any disadvantageous limitations to the present disclosure. The present disclosure has been described in detail herein, and embodiments are also disclosed herein. It will be apparent to those skilled in the art that various changes and modifications may be made to the embodiments disclosed herein without departing from the spirit and scope disclosed herein.

### Example 1

### Synthesis route:

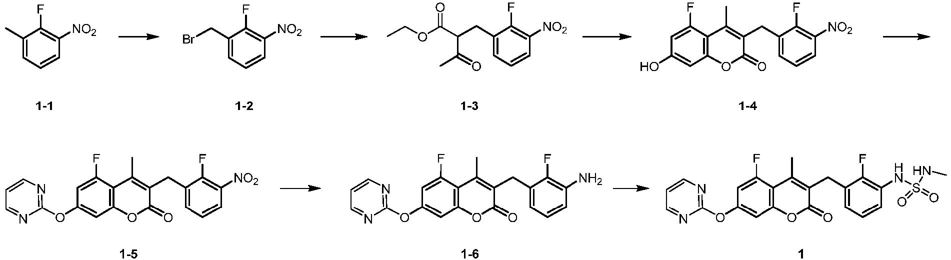

### Step 1: Synthesis of compound 1-2

Compound **1-1** (50 g) was added to glacial acetic acid (500 mL). N-bromosuccinimide (68.84 g) and benzoyl peroxide (2.76 g) were added in sequence. The reaction system was stirred at 85°C for 12 hours. After the reaction was completed, the reaction solution was concentrated to dryness. Water (300 mL) was added, and the mixture was extracted with ethyl acetate (200 mL*3). The layers were separated. The organic phases were combined and washed with saturated brine (150 mL*3). The organic phase was dried over anhydrous sodium sulfate, and filtered. The filtrate was concentrated to obtain a crude product, which was separated and purified by column chromatography (eluent: petroleum ether/ethyl acetate = 100:0-70:30) to obtain compound **1-2.**

The characterization of compound **1-2** is as follows: ¹H NMR (400 MHz, CDCl₃) *δ*: 8.07 - 7.99 (m, 1H), 7.76 - 7.69 (m, 1H), 7.34 - 7.28 (m, 1H), 4.58 - 4.53 (m, 2H).

### Step 2: Synthesis of compound 1-3

60% pure sodium hydride (512.72 mg) was added to anhydrous tetrahydrofuran (15 mL) under nitrogen at 0°C, and ethyl acetoacetate (1.67 g) was slowly added dropwise. The reaction system was stirred at 0°C for 0.5 hours. The above reaction solution was added to a solution of compound **1-2** (3 g) in anhydrous tetrahydrofuran (15 mL) while maintaining the temperature at 0°C. After the addition was completed, the reaction system was heated to 20°C and stirred for 12 hours. After the reaction was completed, the reaction system was quenched by adding saturated aqueous ammonium chloride solution (30 mL). The mixture was extracted with ethyl acetate (30 mL*3), and the layers were separated. The organic phases were combined, dried with anhydrous sodium sulfate, and filtered. The filtrate was concentrated under reduced pressure to obtain a crude product, which was purified by column chromatography (eluent: petroleum ether/ethyl acetate = 100:0-80:20) to obtain compound **1-3.**

The characterization of compound **1-3:** LCMS: m/z (ESI) = 284.0 [M+H]⁺. ¹H NMR (400 MHz, CDCl₃) *δ*: 7.95 - 7.88 (m, 1 H), 7.58 - 7.51 (m, 1 H), 7.23 - 7.16 (m, 1 H), 4.22 - 4.11 (m, 2 H), 3.86 - 3.66 (m, 1 H), 3.33 - 3.18 (m, 2 H), 2.27 (s, 3 H), 1.22 (t, J=7.2 Hz, 3 H).

### Step 3: Synthesis of compound 1-4

Compound **1-3** (600 mg) was dissolved in 70% pure aqueous sulfuric acid solution (6 mL). 5-Fluororesorcinol (271.35 mg) was added, and the reaction system was stirred at 25°C for 16 hours. After the reaction was completed, the reaction system was directly filtered. The filter cake was rinsed with water (10 mL), and the solid was concentrated to dryness under reduced pressure. The solid was purified by column chromatography (eluent: dichloromethane/methanol = 100:0-98:2) to obtain compound **1-4.**

The characterization of compound **1-4:** LCMS: m/z (ESI) =348.0 [M+H]⁺. ¹H NMR (400 MHz, DMSO-*d*₆) *δ*: 10.97-10.77 (m, 1 H), 7.99 (t, J=7.2 Hz, 1 H), 7.57 (t, J=7.2 Hz, 1 H), 7.32 (t, J=8.0 Hz, 1 H), 6.69 - 6.56 (m, 2 H), 4.03 (s, 2 H), 2.47 (s, 3 H).

### Step 4: Synthesis of compound 1-5

Compound **1-4** (100 mg) was dissolved in N,N-dimethylformamide (2.5 mL). Cesium carbonate (112.59 mg) and 2-bromopyrimidine (183.12 mg) were added, and the reaction system was stirred and reacted at 80°C for 1 hour. After the reaction was completed, saturated brine (5 mL) was added to the system, and the mixture was extracted with ethyl acetate (5 mL*3). The layers were separated. The organic phases were combined, dried over anhydrous sodium sulfate, and filtered. The filtrate was concentrated under reduced pressure to obtain a crude product, which was separated by column chromatography (eluent: dichloromethane/methanol = 100:0-98:2) to obtain compound **1-5.**

The characterization of compound **1-5:** LCMS: m/z (ESI) =426.1 [M+H]⁺. ¹H NMR (400 MHz, DMSO-*d*₆) *δ*: 8.71 (d, J=4.4 Hz, 2 H), 8.00 (t, J=7.2 Hz, 1 H), 7.63 (t, J=7.2 Hz, 1 H), 7.41 - 7.25 (m, 4 H), 4.10 (s, 2 H), 2.56 (d, J=6.0 Hz, 3 H).

### Step 5: Synthesis of compound 1-6

Compound **1-5** (35 mg) was dissolved in ethyl acetate (2 mL). Tin dichloride dihydrate (74.27 mg) was added, and the reaction system was stirred and reacted at 80°C for 4 hours. After the reaction was completed, the system was quenched by adding saturated sodium bicarbonate aqueous solution (5 mL), and extracted with ethyl acetate (5 mL*3). The layers were separated. The organic phases were combined, and filtered. The filtrate was concentrated under reduced pressure to obtain compound **1-6.**

The characterization of compound **1-6:** LCMS: m/z (ESI) =396.0 [M+H]⁺. ¹H NMR (400 MHz, DMSO-*d*₆) *δ*: 8.71 (d, J=4.8 Hz, 2 H), 7.37 (t, J=4.8 Hz, 1 H), 7.31 - 7.24 (m, 2 H), 6.78 - 6.68 (m, 1 H), 6.66 - 6.57 (m, 1 H), 6.28 - 6.26 (m, 1 H), 5.08 (s, 2 H), 3.93 (s, 2 H), 2.51 - 2.49 (m, 3 H).

### Step 6: Synthesis of compound 1

Compound **1-6** (40 mg) was dissolved in N,N-dimethylformamide (2 mL). Pyridine (80.03 mg) and a solution of methylaminosulfonyl chloride (98.32 mg) in acetonitrile (1 mL) were added, and the reaction system was stirred at 20°C for 2 hours. After the reaction was completed, the system was quenched by adding saturated brine (5 mL), and extracted with ethyl acetate (5 mL*5). The layers were separated. The organic phases were combined, dried over anhydrous sodium sulfate, and filtered. The filtrate was concentrated under reduced pressure to obtain a crude product. The crude product was separated and purified by preparative high-performance liquid chromatography (high-performance liquid preparative method: Phenomenex preparative chromatograph; column: C18 75*30mm*3µm; mobile phase A: 10 mM aqueous ammonium bicarbonate solution (containing 0.05% ammonia water), mobile phase B: acetonitrile; running gradient: B%: 25%-60% in 8 min) to obtain compound **1.**

The characterization of compound **1:** LCMS: m/z (ESI) =489.1 [M+H]⁺. ¹H NMR (400 MHz, DMSO-*d*₆) *δ*: 9.36 (br s, 1 H), 8.71 (d, J=4.8 Hz, 2 H), 7.37 (t, J=4.8 Hz, 1 H), 7.32 - 7.25 (m, 3 H), 7.22-7.20 (m, 1 H), 7.04-7.02 (m, 1 H), 6.95 - 6.92 (m, 1 H), 3.99 (s, 2 H), 2.57 - 2.51 (m, 6 H).

### Example 2

### Synthesis route:

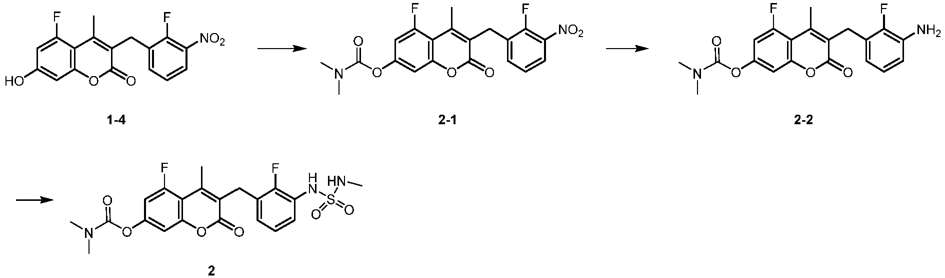

### Step 1: Synthesis of compound 2-1

Compound **1-4** (100 mg) was dissolved in N,N-dimethylformamide (2.5 mL). Potassium carbonate (119.39 mg) was added. The mixture was stirred at 0°C for 0.5 hours, and then dimethylaminoformyl chloride (37.16 mg) was added. The mixture was stirred and reacted at 20°C for 2 hours. After the reaction was completed, saturated brine (5 mL) was added to the system, and the mixture was extracted with ethyl acetate (5 mL*3). The layers were separated. The organic phases were combined, dried over anhydrous sodium sulfate, and filtered. The filtrate was concentrated under reduced pressure to obtain a crude product, which was separated by column chromatography (eluent: petroleum ether/ethyl acetate = 100:0-60:40) to obtain compound **2-1.**

The characterization of compound **2-1:** LCMS: m/z (ESI) =419.1 [M+H]⁺. ¹H NMR (400 MHz, DMSO-*d*₆) *δ*: 8.07-8.00 (m, 1 H), 7.68-7.62 (m, 1 H), 7.34 - 7.27 (m, 1 H), 7.21 - 7.14 (m, 2 H), 4.08 (s, 2 H), 3.05 (s, 3 H), 2.93 (s, 3 H), 2.54 (d, *J*=6.0 Hz, 3 H).

### Step 2: Synthesis of compound 2-2

Compound **2-1** (100 mg) was dissolved in ethyl acetate (5 mL). Tin dichloride dihydrate (215.75 mg) was added. The reaction system was stirred and reacted at 80°C for 4 hours. After the reaction was completed, the system was quenched by adding saturated aqueous sodium bicarbonate solution (5 mL), and extracted with ethyl acetate (5 mL*3). The layers were separated. The organic phases were combined, dried over anhydrous sodium sulfate, and filtered. The filtrate was concentrated under reduced pressure to obtain compound **2-2.**

The characterization of compound **2-2:** LCMS: m/z (ESI) =389.0 [M+H]⁺. ¹H NMR (400 MHz, DMSO-*d*₆) *δ*: 7.19 - 7.11 (m, 2 H), 6.75 - 6.68 (m, 1 H), 6.65 - 6.56 (m, 1 H), 6.33 - 6.24 (m, 1 H), 5.08 (s, 2 H), 3.91 (s, 2 H), 3.05 (s, 3 H), 2.93 (s, 3 H), 2.56 - 2.47 (m, 3 H).

### Step 3: Synthesis of compound 2

Compound **2-2** (100 mg) was dissolved in N,N-dimethylformamide (3 mL). Pyridine (203.67 mg) and a solution of methylaminosulfonyl chloride (250.21 mg) in acetonitrile (1.5 mL) were added, and the reaction system was stirred at 20°C for 2 hours. After the reaction was completed, the system was quenched by adding saturated brine (15 mL), and extracted with ethyl acetate (15 mL*5). The layers were separated. The organic phases were combined, dried over anhydrous sodium sulfate, and filtered. The filtrate was concentrated under reduced pressure to obtain a crude product. The crude product was separated and purified by preparative high-performance liquid chromatography (high-performance liquid preparative method: Phenomenex preparative chromatograph; column: C18 75*30mm*3µm; mobile phase A: 10 mM aqueous ammonium bicarbonate solution (containing 0.05% ammonia water), mobile phase B: acetonitrile; running gradient: B%: 25%-60% in 8 min), to obtain compound 2.

The characterization of compound 2: LCMS: m/z (ESI) =482.1 [M+H]⁺. ¹H NMR (400 MHz, CDCl₃) *δ*: 7.41 (t, J=8.0 Hz, 1 H), 7.03 (t, J=8.0 Hz, 1 H), 7.00 - 6.87 (m, 3 H), 6.60 (s, 1 H), 4.44 - 4.35 (m, 1 H), 4.08 (s, 2 H), 3.12 (s, 3 H), 3.04 (s, 3 H), 2.77 (d, J=5.2 Hz, 3 H), 2.56 (d, J=6.0 Hz, 3 H).

### Example 3

### Synthesis route:

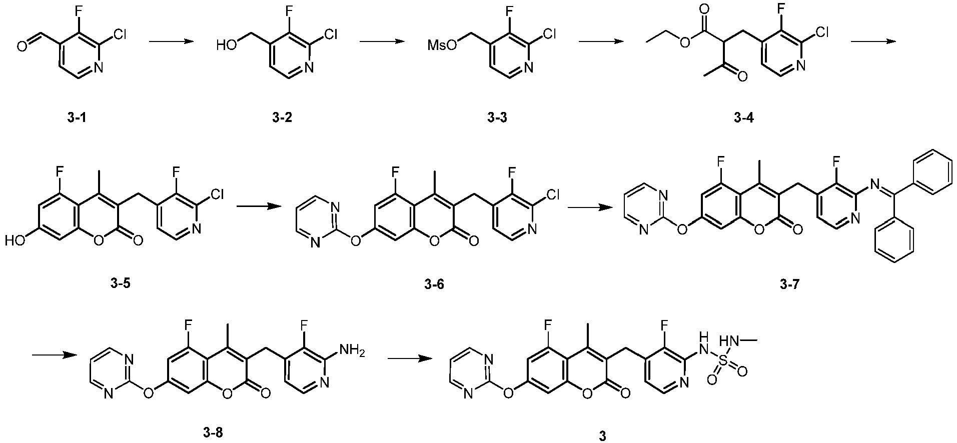

### Step 1: Synthesis of compound 3-2

Compound **3-1** (41 g) was dissolved in anhydrous methanol (410 mL). Sodium borohydride (10.77 g) was added in batches at 0°C. The mixture was stirred at 20°C under nitrogen for 2 hours. Acetone (50 mL) was slowly added dropwise to the reaction solution under nitrogen flow. The mixture was stirred for 1 hour. The mixture was concentrated under reduced pressure to dryness to remove the solvent. Ethyl acetate (500 mL), saturated brine (200 mL) and water (200 mL) were added to the crude product. The layers were separated. The organic phases were combined, dried over anhydrous sodium sulfate, and then filtered. The filtrate was concentrated under reduced pressure to dryness to remove the solvent to obtain compound **3-2.**

The characterization of compound **3-2** is as follows: LCMS: m/z (ESI) =161.9 [M+H]⁺. ¹H NMR (400 MHz, CDCl₃) *δ*: 8.18 (d, J=4.8 Hz, 1 H), 7.47 (t, J=4.8 Hz, 1 H), 4.86 (s, 2 H), 2.77 (s, 1 H).

### Step 2: Synthesis of compound 3-3

Compound **3-2** (10 g) was dissolved in anhydrous tetrahydrofuran (100 mL), and then triethylamine (12.53 g) was added. Methylsulfonic anhydride (12.94 g) was added in batches, and the mixture was stirred at 20 °C for 1 hour. The mixture was concentrated under reduced pressure to dryness to remove the solvent, and the crude product was purified by silica gel column chromatography (gradient elution: petroleum ether/ethyl acetate = 100:0-70:30) to obtain compound **3-3.**

The characterization of compound **3-3** is as follows: LCMS: m/z (ESI) =239.9 [M+H]⁺. ¹H NMR (400 MHz, CDCl₃), *δ*: 8.28 (d, J=4.8 Hz, 1 H), 7.40 (t, J=4.8 Hz, 1 H), 5.34 (s, 2 H), 3.13 (s, 3 H).

### Step 3: Synthesis of compound 3-4

Sodium iodide (625.45 mg) and ethyl acetoacetate (1.09 g) were dissolved in anhydrous tetrahydrofuran (10 mL), and a solution of lithium tert-butoxide in tetrahydrofuran (2.2 M, 2.09 mL) was added dropwise. After the addition was completed, the mixture was stirred for 1 hour, and then cooled to 0°C. A solution of compound **3-3** (1 g) in anhydrous tetrahydrofuran (5 mL) was added under nitrogen. After the addition was completed, the mixture was stirred at 20°C for 1 hour. Saturated brine (20 mL), water (20 mL), and ethyl acetate (20 mL) were added, and the layers were separated. The organic phase was dried over anhydrous sodium sulfate and filtered. The filtrate was concentrated under reduced pressure to dryness to remove the solvent. The crude product was purified by alumina column chromatography (gradient elution: petroleum ether/ethyl acetate = 100:0-90:10) to obtain compound **3-4.**

The characterization of compound **3-4** is as follows: LCMS: m/z (ESI) =273.9 [M+H] ⁺.

### Step 4: Synthesis of compound 3-5

Compound **3-4** (0.11 g) was dissolved in 98% concentrated sulfuric acid (788.39 mg). The solution was cooled to 0°C, and 5-fluororesorcinol (51.49 mg) was added. The mixture was stirred at 20°C for 16 hours. After the reaction was completed, ethyl acetate (5 mL), saturated brine (3 mL), and water (3 mL) were added to the reaction solution, and the layers were separated. The organic phase was dried over anhydrous sodium sulfate and filtered. The filtrate was concentrated under reduced pressure to dryness to remove the solvent. The crude product was purified by silica gel column chromatography (gradient elution: petroleum ether/ethyl acetate = 100:0-0:100) to obtain compound **3-5.**

The characterization of compound **3-5** is as follows: LCMS: m/z (ESI) =337.9 [M+H] ⁺. ¹H NMR (400 MHz, CDCl₃) *δ*: 11.00 (s, 1 H), 8.13-8.11 (d, J=5.2 Hz, 1 H), 7.30-7.20 (t, J=5.2 Hz, 1 H), 6.69 - 6.51 (m, 2 H), 4.02 (s, 2 H), 2.50-2.45 (d, J=6.0 Hz, 3 H).

### Step 5: Synthesis of Compound 3-6

Compound **3-5** (310 mg) was dissolved in N,N-dimethylformamide (3 mL). Cesium carbonate (358.91 mg) and 2-bromopyrimidine (175.13 mg) were added, and the mixture was stirred at 80°C for 8 hours. Ethyl acetate (20 mL) and water (20 mL) were added to the reaction solution, and the layers were separated. The organic phase was dried over anhydrous sodium sulfate and filtered. The filtrate was concentrated under reduced pressure to dryness to remove the solvent. The crude product was purified by silica gel column chromatography (gradient elution: petroleum ether/ethyl acetate = 100:0-70:30) to obtain compound **3-6.**

The characterization of compound **3-6** is as follows: LCMS: m/z (ESI) =416.0 [M+H] ⁺. ¹H NMR (400 MHz, CDCl₃) *δ*: 8.63 (d, J=4.8 Hz, 2 H), 8.10 (d, J=4.8 Hz, 1 H), 7.18-7.14 (m, 2 H), 7.12 - 7.07 (m, 1 H), 6.96 (m, 1 H), 4.13 (s, 2 H), 2.61 (d, J=6.0 Hz, 3 H).

### Step 6: Synthesis of compound 3-7

Compound **3-6** (140 mg), benzophenone imine (82.47 mg), tris(dibenzylacetone)dipalladium (30.83 mg), 4,5-bis(diphenylphosphino)-9,9-dimethylxanthene (38.97 mg), and cesium carbonate (329.13 mg) were dissolved in anhydrous toluene (5 mL) and the mixture was stirred at 110 °C for 3 hours under nitrogen. The mixture was concentrated under reduced pressure to dryness to remove the solvent, and the crude product was purified by silica gel column chromatography (gradient elution: petroleum ether/ethyl acetate = 100:0-50:50) to obtain compound **3-7.**

The characterization of compound **3-7** is as follows: LCMS: m/z (ESI) =561.2 [M+H] ⁺. ¹H NMR (400 MHz, CDCl₃) *δ*: 8.63 (d, J=4.8 Hz, 2 H), 8.02 (d, J=5.2 Hz, 1 H), 7.47 - 7.28 (m, 9 H), 7.17 (t, J=4.8 Hz, 2 H), 7.07 (s, 1 H), 6.98 - 6.92 (m, 1 H), 6.81 (s, 1 H), 3.94 (s, 2 H), 2.30 (m, 3 H).

### Step 7: Synthesis of compound 3-8

Compound **3-7** (130 mg) was dissolved in anhydrous tetrahydrofuran (5.2 mL), and then 1 M dilute hydrochloric acid (260.00 µL) was added. The mixture was stirred at 20°C for 2 hours. Ethyl acetate (10 mL), saturated brine (5 mL) and water (5 mL) were added to the reaction solution, and the layers were separated. The aqueous phase was collected and adjusted to pH 9 with saturated sodium carbonate solution, and then ethyl acetate (10 mL) was added. The layers were separated. The organic phase was dried over anhydrous sodium sulfate and filtered. The filtrate was concentrated under reduced pressure to dryness to remove the solvent to obtain compound **3-8.**

The characterization of compound **3-8** is as follows: LCMS: m/z (ESI) =397.0 [M+H] ⁺. ¹H NMR (400 MHz, CDCl₃) *δ*: ppm 8.63 (d, J=4.8 Hz, 2 H), 7.70 (d, J=5.4 Hz, 1 H), 7.17 (t, J=4.8 Hz, 1 H), 7.11 - 7.02 (m, 1 H), 7.00 - 6.90 (m, 1 H), 6.53 (t, J=5.4 Hz, 1 H), 5.19 - 5.01 (m, 2 H), 4.07 (s, 2 H), 2.58 (d, J=6.0 Hz, 3 H).

### Step 8: Synthesis of compound 3

Compound **3-8** (103 mg) was dissolved in N,N-dimethylformamide (1.2 mL). Pyridine (113.06 mg) and a solution of methylaminosulfonyl chloride (101.01 mg) in acetonitrile (0.6 mL) were added. The mixture was stirred at 20°C for 2 hours. The reaction solution was separated and purified by preparative HPLC (HPLC preparative method: Waters Xbridge BEH preparative chromatograph; column: C18 100*30mm*10µm; mobile phase A: 0.1% aqueous ammonium bicarbonate solution, mobile phase B: acetonitrile; running gradient: B%: 25%-55% in 8 min) to obtain compound **3.**

The characterization of compound **3** is as follows: LCMS: m/z (ESI) =490.0 [M+H] ⁺. ¹H NMR (400 MHz, DMSO-*d*₆) *δ*: ppm 8.71 (d, J=4.8 Hz, 2 H), 7.87 (d, J=5.0 Hz, 1 H), 7.44 - 7.23 (m, 3 H), 6.80 - 6.60 (m, 1 H), 4.00 (s, 2 H), 2.53 (m, 3 H), 2.48 (s, 3 H).

### Example 4

### Synthesis route:

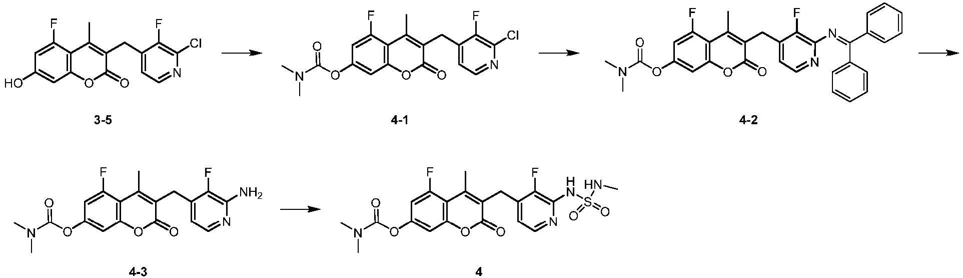

### Step 1: Synthesis of compound 4-1

Compound **3-5** (300 mg) was dissolved in N,N-dimethylformamide (3 mL) under nitrogen, and potassium carbonate (368.34 mg) was added. Dimethylaminoformyl chloride (124.19 mg) was added at 0°C. The mixture was stirred at 20°C for 3 hours. Water (6 mL) was added to the reaction solution, and the mixture was stirred for 30 minutes and filtered. The filter cake was rinsed with water (2 mL), collected, and dried by rotary evaporation under vacuum to obtain compound **4-1.**

The characterization of compound **4-1** is as follows: LCMS: m/z (ESI) =409.0 [M+H]⁺. ¹H NMR (400 MHz, DMSO-*d*₆) *δ*: 8.13 (d, *J*=5.0 Hz, 1 H), 7.32 (s, 1 H), 7.25 - 7.10 (m, 2 H), 4.08 (s, 2 H), 3.05 (s, 3 H), 2.93 (s, 3 H), 2.53 (d, *J*=6.0 Hz, 3 H).

### Step 2: Synthesis of compound 4-2

Compound **4-1** (360 mg), benzophenone imine (215.47 mg), tris(dibenzylacetone)dipalladium (80.64 mg), 4,5-bis(diphenylphosphino)-9,9-dimethylxanthene (101.91 mg), and cesium carbonate (860.81 mg) were dissolved in toluene (5 mL). The mixture was stirred at 110 °C for 3 hours under nitrogen. The mixture was concentrated under reduced pressure to dryness to remove the solvent, and the crude product was purified by silica gel column chromatography (gradient elution: petroleum ether/ethyl acetate = 100:0-50:50) to obtain compound **4-2.**

The characterization of compound **4-2** is as follows: LCMS: m/z (ESI) =554.2 [M+H]⁺. ¹H NMR (400 MHz, CDCl₃) *δ*: 8.01 (d, *J*=5.0 Hz, 1 H), 7.91 - 7.72 (m, 2 H), 7.53 - 7.32 (m, 4 H), 7.23 - 7.06 (m, 4 H), 7.00-6.97 (m, 1 H), 6.95-6.90 (m, 1 H), 6.80 (t, *J*=5 Hz, 1 H), 3.92 (s, 2 H), 3.13 (s, 3 H), 3.05 (s, 3 H), 2.27 (d, *J*=6.0 Hz, 3 H).

### Step 3: Synthesis of compound 4-3

Compound **4-2** (190 mg) was dissolved in anhydrous tetrahydrofuran (7.6 mL), and then 1M dilute aqueous hydrochloric acid solution (384.80 µL) was added . The mixture was stirred at 20 °C for 2 hours. The reaction solution was directly filtered, and the filter cake was rinsed with anhydrous tetrahydrofuran (0.5 mL). The filter cake was dried by rotary evaporation under vacuum to obtain compound **4-3** hydrochloride.

The characterization of compound **4-3** is as follows: LCMS: m/z (ESI) =390.0 [M+H] ⁺. ¹H NMR (400 MHz, DMSO-*d*₆) *δ*: 7.65 (d, *J*=6.0 Hz, 1 H), 7.26 - 7.12 (m, 2 H), 6.60 (s, 1 H), 4.03 (s, 2 H), 3.05 (s, 3 H), 2.93 (s, 3 H), 2.53 (s, 3 H).

### Step 4: Synthesis of compound 4

Compound **4-3** hydrochloride (98 mg) was dissolved in N,N-dimethylformamide (1 mL). Pyridine (100.13 mg) and a solution of methylaminosulfonyl chloride (89.46 mg) in acetonitrile (0.5 mL) were added. The mixture was stirred at 20°C for 2 hours. The reaction solution was separated and purified by preparative HPLC (preparative HPLC method: Phenomenex preparative chromatograph; column: C18 75*30mm*3µm; mobile phase A: 0.1% aqueous ammonium bicarbonate solution, mobile phase B: acetonitrile; running gradient: B%: 25%-45% in 8 min) to obtain compound **4.**

The characterization of compound **4** is as follows: LCMS: m/z (ESI) =483.2 [M+H] ⁺. ¹H NMR (400 MHz, DMSO-*d*₆) *δ*: 7.80 (d, *J*=4.6 Hz, 1 H), 7.23 - 7.06 (m, 2 H), 6.62 (s, 1 H), 3.97 (s, 2 H), 3.06 (s, 3 H), 2.94 (s, 3 H), 2.55 - 2.51 (m, 3 H), 2.45 (s, 3 H).

### Evaluation System

### Assay Example 1: Evaluation of HCT116 cytological activity in vitro

### Assay Materials:

McCoy's 5A medium, penicillin/streptomycin antibiotics purchased from Vicente, fetal bovine serum purchased from Biosera. 3D CellTiter-Glo (cell viability chemiluminescence detection reagent) reagent purchased from Promega. HCT116 cell line purchased from Nanjing Kebai Biotechnology Co., Ltd. Envision multi-label analyzer (PerkinElmer).

### Assay method:

HCT116 cells were seeded in ultra-low attachment 96-well U-shaped plates, with 80 µL of cell suspension per well, containing 1000 HCT116 cells. The cell plate was cultured in a carbon dioxide incubator overnight.

The compound to be tested was serially diluted 5-fold to the 9th concentration using a pipette, that is, from 2 mM to 5.12nM, in duplicates. 78 µL of culture medium was added to a middle plate, and then 2 µL of the serially diluted compound per well was transferred to the middle plate according to the corresponding position. After mixing well, 20 µL of the mixture was transferred to each well of the cell plate. The concentration range of the compound transferred to the cell plate was 10 µM to 0.0256nM. The cell plate was placed in a carbon dioxide incubator and cultured for 5 days. Another cell plate was prepared, and its signal value was read on the day of drug addition as the maximum value (Max value in the equation below) for data analysis.

100 µL of cell viability chemiluminescent detection reagent was added to the cell plate and the mixture was incubated at room temperature for 10 minutes to stabilize the luminescent signal. The plate was read using a multi-label analyzer.

### Data analysis:

The raw data was converted into inhibition rate using the equation (Sample-Min)/(Max-Min)*100%, and the IC₅₀ value may be obtained by four-parameter curve fitting (obtained in the "log(inhibitor) vs. response -- Variable slope" mode in GraphPad Prism). The assay results of the inhibitory activity of the compounds of the present disclosure on HCT116 cell proliferation are shown in Table 1.

**Table 1: Assay results of in vitro screening of the compounds of the present disclosure**

| **Compound No.** | **HCT116/ IC₅₀ (nM)** |
|---|---|
| **1** | 17.0 |
| **2** | 7.2 |
| **3** | 28.7 |
| **4** | 4.6 |

| | |
|---|---|
| Conclusion: The compounds of the present disclosure exhibit good inhibitory activity on cell proliferation in the HCT116 cell line. | |

### Assay Example 2: In vivo pharmacokinetic study of compound 3

Pharmacokinetic study of the test compound in CD-1 male mice after oral and intravenous injection

The test compound was mixed with 10% dimethyl sulfoxide/30% PEG-400/60% aqueous solution, vortexed and sonicated to prepare a 0.20 mg/mL clear solution. The clear solution was filtered through a microporous filter for later use. Male CD-1 mice aged 7 to 10 weeks were selected and intravenously injected with a candidate compound solution and a reference compound VS-6766 solution. The reference compound and the test compound were respectively mixed with 20% sulfobutyl-β-cyclodextrin/80% aqueous solution, vortexed and sonicated to prepare a 0.20 mg/mL nearly transparent solution containing fine particles, and the candidate compound solution was orally administered. Whole blood was collected for a certain period of time, and the plasma was prepared. The drug concentration was analyzed by LC-MS/MS method, and the pharmacokinetic parameters were calculated using Phoenix WinNonlin software (Pharsight, USA). The assay results are shown in Table 2:

**Table 2. Pharmacokinetic results of the test compound**

| Route of administration | Pharmacokinetic parameters | **Compound 3** |
|---|---|---|
| Intravenous injection | Dose(mg/kg) | 1.04 |
| | Half-life T_{1/2} (h) | 5.73 |
| | Clearance CL (ml/min/kg) | 0.69 |
| | Apparent volume of distribution Vdₛₛ (L/kg) | 0.35 |
| | Area under the plasma concentration-time curve AUC₀₋ₗₐₛₜ | 46836 |
| Oral administration | Dose(mg/kg) | 2.16 |
| | Time to peak Tₘₐₓ (h) | 0.63 |
| | Peak concentration Cₘₐₓ | 10895 |
| | Area under the plasma concentration-time curve AUC₀₋ₗₐₛₜ | 85836 |
| | Bioavailability F (%) | 89.8% |

Assay conclusion: PK studies show that the compound of the present disclosure has higher unbound plasma exposure and good oral bioavailability in mice.

### Assay Example 3: In vivo pharmacodynamic study of compound 3

In vivo pharmacodynamic study of the test drug on the subcutaneous xenograft tumor model of human non-small cell lung cancer NCI-H358 in female BALB/c Nude mice
**1. Cell culture:** Human lung cancer cells NCI-H358 (ECACC-95111733) were revived and cultured in vitro in monolayers in RPMI 1640 medium with 10% fetal bovine serum and 1% double antibody. The cells were cultured in a 37°C 5% CO₂ cell incubator, and passaged twice a week. When the saturation of NCI-H358 cells reached 80%-90%, the cells were harvested, counted, and inoculated.
**2. Animals:** BALB/c Nude mice, female, 6-8 weeks old, weighing 18-22 g
**3. Tumor inoculation:** 5×10⁶ NCI-H358 cells were inoculated on the right side of the neck of each mouse, with an inoculation volume of 0.1 mL. The cell suspension was a mixture of PBS and Matrigel (3:1). When the average tumor volume reached 150-200 mm³, the mice were randomly divided into groups for drug administration. The drug administration scheme and the number of assay animals in each group are shown in Table 3 below: Assay animal grouping and administration regimen

**Table 3. Assay animal grouping and administration regimen**

| **Group** | **N** | **Treatment** | **Dosage (mg/kg)** | **Dosing volume parameters (µL/g)** | **Route of administration** | **Dosing frequency** |
|---|---|---|---|---|---|---|
| **1** | **6** | **Vehicle (control group)** | **--** | **10** | **p.o.** | **QD*28 days** |
| **3** | **6** | **Compound 3** | **0.3** | **10** | **p.o.** | **QD*28 days (Discontinuing during D10-D17) (Discontinuing during D23-D25)** |
| **4** | **6** | **Compound 3** | **1.5** | **10** | **p.o.** | **QD*28 days (Discontinuing during D3-D13) (Changed to 0.05 after D14)** |

| | | | | | | |
|---|---|---|---|---|---|---|
| Note: QD means once daily. | | | | | | |

### 4. Tumor measurements and assay parameters

The assay index is to examine whether the tumor growth is inhibited, delayed or cured. The tumor diameter was measured with a vernier caliper twice a week. The calculation formula of tumor volume is: V = 0.5 *a* × *b*², where *a* and *b* represent the long diameter and short diameter of the tumor respectively.

The anti-tumor efficacy of the compound was evaluated by TGI (%) or relative tumor proliferation rate T/C (%). TGI (%) reflects the tumor growth inhibition rate. Calculation of TGI (%): TGI (%) = [1- (average tumor volume of a certain treatment group at a certain measurement - average tumor volume of the treatment group at grouping) / (average tumor volume of the vehicle control group at the same measurement - average tumor volume of the vehicle control group at grouping)] × 100%. Relative tumor proliferation rate T/C (%): The calculation formula is as follows: T/C % = T_{RTV} / C_{RTV} × 100 % (T_{RTV}: RTV of the treatment group; C_{RTV}: RTV of the negative control group). The relative tumor volume (RTV) was calculated based on the results of tumor measurement. The calculation formula is RTV = Vₜ/V₀, where V₀ is the average tumor volume measured at grouping (i.e. PG-D0), Vₜ is the average tumor volume at a certain measurement, and T_{RTV} and C_{RTV} are taken from the same day. TGI (%) reflects the tumor growth inhibition rate. TGI (%) = [(1 - (average tumor volume at the end of a treatment group's administration - average tumor volume at the beginning of the treatment group's administration))/(average tumor volume at the end of the vehicle control group's treatment - average tumor volume at the beginning of the vehicle control group's treatment)] × 100%.

### 5. Assay results

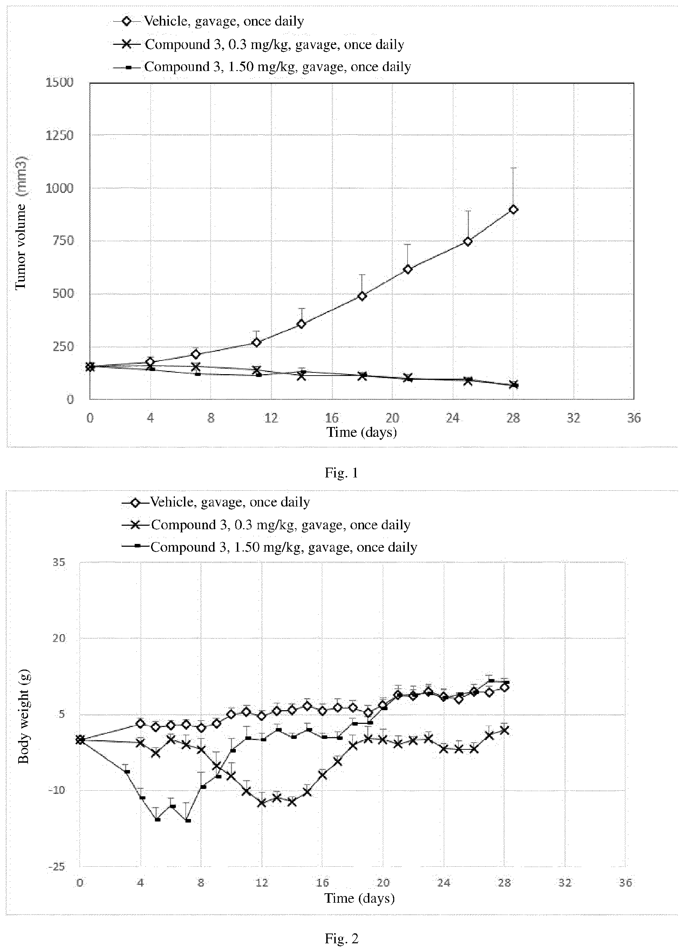

The assay results are shown in Figs. 1 and 2.

The results after 28 days of administration are shown in Table 4

**Table 4. T/C and TGI on the 28th day after administration**

| Compound | Dosage | Mean tumor volume | T/C | TGI |
|---|---|---|---|---|
| Vehicle | N/A | 899 mm³ | N/A | N/A |
| Compound 3 | 0.3 mg/kg | 71 mm³ | 7.90% | 111.4% |
| Compound 3 | 1.5 mg/kg | 67 mm³ | 7.45% | 111.9% |

Assay conclusion: The compound of the present disclosure has significant tumor-suppressing effects. The current dosage and intermittent administration may have achieved the maximum tumor-suppressing effect of this target. Future in vivo efficacy studies will consider reducing the dosage or adopting intermittent administration to demonstrate dose-dependency and improve animal tolerance.

## Claims

1. A compound represented by formula (I) or a pharmaceutically acceptable salt thereof 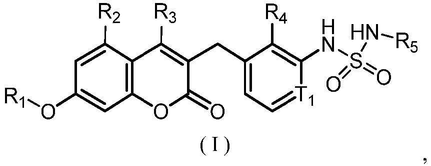 wherein
T₁ is selected from CH and N;
R₁ is selected from pyrimidinyl and -C(=O)-C₁₋₄ alkylamino;
R₂ is halogen;
R₃ is selected from halogen, C₁₋₃ alkyl, -CH₂-C₁₋₄ alkylamino and -CH₂-4- to 6-membered N-containing heterocycloalkyl, wherein the C₁₋₃ alkyl, -CH₂-C₁₋₄ alkylamino and -CH₂-4- to 6-membered N-containing heterocycloalkyl are each independently optionally substituted with 1, 2 or 3 halogens;
R₄ is selected from halogen and C₁₋₃ alkyl, wherein the C₁₋₃ alkyl is optionally substituted with 1, 2 or 3 halogens;
R₅ is C₁₋₃ alkyl, wherein the C₁₋₃ alkyl is optionally substituted with 1, 2 or 3 halogens.

2. The compound according to claim 1 or a pharmaceutically acceptable salt thereof, wherein R₁ is selected from

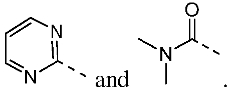

3. The compound according to claim 1 or a pharmaceutically acceptable salt thereof, wherein R₂ is F.

4. The compound according to claim 1 or a pharmaceutically acceptable salt thereof, wherein R₃ is selected from F, Cl, Br, CH₃, CH₂CH₃, CH₂NHCH₃ and CH₂N(CH₃)₂, wherein the CH₃, CH₂CH₃, CH₂NHCH₃ and CH₂N(CH₃)₂ are optionally substituted with 1, 2 or 3 halogens.

5. The compound according to claim 1 or 4 or a pharmaceutically acceptable salt thereof, wherein R₃ is selected from CH₃ and CH₂N(CH₃)₂.

6. The compound according to claim 1 or a pharmaceutically acceptable salt thereof, wherein R₄ is F.

7. The compound according to claim 1 or a pharmaceutically acceptable salt thereof, wherein R₅ is CH₃.

8. A compound represented by the following formula or a pharmaceutically acceptable salt thereof,
